# EUROPEAN PATENT APPLICATION

(11) **EP 1 445 716 A1**
(43) Date of publication of application: **11.08.2004**
(21) Application number: 02802054.3
(22) Date of filing: 22.10.2002
(51) Int. Cl.: G06F 17/60

(54) **HEALTH MANAGEMENT SYSTEM AND HEALTH MANAGEMENT PROGRAM**

(30) Priority: 23.10.2001 JP 2001325560
(71) Applicant: Citizen Watch Co. Ltd., Tokyo 188-8511 (JP)
(72) Inventor: SHIMIZU, Hideki, Nishitokyo-shi, Tokyo 188-8511 (JP); TSUKAMOTO, Yasushi, NIshitokyo-shi, Tokyo 188-8511 (JP)
(74) Representative: Mounteney, Simon James
(86) International application number: PCT/JP2002/010963
(87) International publication number: WO 2003/036539

(57) **Abstract**

A healthcare apparatus (1) comprises input means (b1-bn, e1-en, 10, 13) for inputting a plurality of types of data to be used for the healthcare or diagnosis of a subject, storage means (19) for storing the data, and analyzing means (21) for analyzing the presence of correlation between the data. The analyzing means (21) analyzes the presence of correlation between the data by determining the correspondence between the transition of the data with time and a plurality of predetermined transition patterns.

## Description

### TECHNICAL FIELD

The present invention relates to a healthcare apparatus and a healthcare program used to detect and analyze the health of a subject, thereby serving for healthcare or diagnosis.

### BACKGROUND ART

In a conventional method of detecting and displaying living body information, such as the temperature, blood pressure, etc. of the human body, only information at the time of measurement is displayed. However, living body information at the time of measurement alone cannot be satisfactory information for healthcare. Described in Japan Patent Application Laid-Open No. 4-354930, therefore, is a health information measuring apparatus that measures the blood pressure, pulse rate, bodily temperature, etc. in a time series and graphically displays the result of the measurement, mean values, maximum values, minims values, etc.

Described in Japan Patent Application Laid-Open No. 3-7136, moreover, is a health determining apparatus that executes fuzzy inference based on given rules and displays the healthiness in accordance with information for the result of an inquiry obtained from inquiry means and the living body information, including the blood pressure, bodily temperature, etc. detected by means of living body information detecting means.

Only measuring and graphically displaying the living body information in a time series and displaying the maximum and minimum values cannot be satisfactory measures to control and diagnose the health. According to the method in which fuzzy inference is executed and the healthiness is determined and displayed by the given rules based on the result information for a predetermined inquiry and the measured living body information, moreover, the healthiness is simply displayed without fulfilling any conditions, and the healthiness is obtained uniformly by the fuzzy inference based on the given rules. In some cases, therefore, the result of determination may be wrong.

### DISCLOSURE OF THE INVENTION

The object of the present invention is to provide a healthcare apparatus and a healthcare program, by which the health of a subject can be determined objectively lest a wrong decision be made.

In order to achieve the above object, a healthcare apparatus according to the present invention comprises input means for inputting a plurality of types of data to be used for the healthcare or diagnosis of a subject, storage means for storing the data, and analyzing means for analyzing the presence of correlation between the data.

The healthcare apparatus according to the present invention may assume the following aspects.

The analyzing means analyzes the presence of correlation between the data by determining the correspondence between the transition of the data with time and a plurality of predetermined transition patterns.

The healthcare apparatus extracts data having a given transition pattern from the aforesaid plurality of types of data.

The given transition pattern can be selected from a plurality of predetermined patterns and assigned.

If data of one type is assigned, data of another type having the same transition pattern with the data of the assigned type is extracted.

If data of one type is assigned, data of another type having the same transition pattern as that of the data of the assigned type and data of a type having a transition pattern associated with the aforesaid transition pattern are extracted.

The associated transition pattern is a transition pattern having a tendency to contradict the transition pattern of the data of the assigned type.

If one transition pattern, among the aforesaid plurality of patterns, is assigned, data having the same transition pattern as the assigned transition pattern and data having a transition pattern associated with the aforesaid transition pattern are extracted.

The associated transition pattern is a transition pattern having a tendency to contradict the assigned transition pattern.

The data of the aforesaid plurality of types include living body data obtained by measuring the state of the body of the subject, environment data obtained by measuring the conditions of the living environment of the subject, life data obtained by quantifying the physical condition and lifestyle of the subject, etc.

A healthcare program according to the present invention realizes a storage function to load a computer with data of a plurality of types used for a plurality of healthcares or diagnoses of a subject and store a storage unit of the computer with the data of the aforesaid plurality of types and an analyzing function to analyze the presence of correlation between the data stored in the storage unit.

The healthcare program according to the present invention may assume the following aspects.

The analyzing function analyzes the presence of correlation between the data by determining the correspondence between the transition of the data and a plurality of predetermined transition patterns.

The healthcare program realizes an extracting function to extract data having a given transition pattern from the data of the aforesaid plurality of predetermined types.

The healthcare program further realizes a function to select the given transition pattern from a plurality of predetermined patterns by means of input means of the computer and assign the pattern.

The healthcare program further realizes a function to extract data of another type having the same transition pattern as that of the data of one type if the data of the one type is assigned by means of input means of the computer.

The healthcare program further extracts data of. another type having the same transition pattern as that of the data of one type and data of a type having a transition pattern associated with the aforesaid transition pattern if the data of the one type is assigned by means of input means of the computer.

The associated transition pattern is a transition pattern having a tendency to contradict the transition pattern of the data of the assigned type.

The healthcare program realizes an extracting function to extract data having the same transition pattern as one transition pattern and data having a transition pattern associated with the aforesaid transition pattern if the one transition pattern is assigned among the aforesaid plurality of transition patterns.

The associated transition pattern is a transition pattern having a tendency to contradict the assigned transition pattern.

The data of the aforesaid plurality of types include living body data obtained by measuring the state of the body of the subject, environment data obtained by measuring the conditions of the living environment of the subject, life body data obtained by quantifying the physical condition and lifestyle of the subject, etc.

According to the present invention, a plurality of types of data to be used for the healthcare or diagnosis of the subject are previously measured and stored so that the respective patterns of various these data can be extracted. Based on the obtained patterns, therefore, the health of the subject can be grasped objectively and automatically.

Since the data having the same pattern as that of the assigned data can be extracted and displayed, the health can be grasped more easily.

Further, the living body data including the weight, bodily temperature, pulse rate, etc., daily life data including meals, evacuation, sleep, etc., environment data, etc. are previously measured and stored, and the daily life data that cannot be measured in numerical values are quantified into objective data. By doing this, the health can be grasped more objectively.

### BRIEF DESCRIPTION OF THE DRAWINGS

FIG. 1 is a block diagram showing a principal part of one embodiment of a healthcare apparatus according to the present invention;
FIG. 2 is a diagram illustrating the operation the healthcare apparatus shown in FIG. 1;
FIG. 3 is a diagram showing an example of an initial picture displayed on display means of the healthcare apparatus shown in FIG. 1;
FIG. 4 shows an example of an analysis setting picture displayed on the display means of the healthcare apparatus shown in FIG. 1;
FIG. 5 is a diagram illustrating five preset examples of transition patterns;
FIG. 6 is a diagram showing an example of a picture that appears when an "inquiry button" is clicked on the picture of FIG. 3;
FIG. 7 is a diagram showing an example of a picture that appears when a "health diary button" is clicked on the picture of FIG. 3;
FIG. 8 shows an operational flow of a first graph analysis executed when a "graph analysis button" is clicked on the picture of FIG. 3;
FIG. 9 shows an operational flow of a second graph analysis executed when the analysis setting picture of FIG. 4 is displayed by clicking a "detail setting button" on the picture of FIG. 3;
FIG. 10 shows an operational flow of a third graph analysis executed when the analysis setting picture of FIG. 4 is displayed by clicking the "detail setting button" on the picture of FIG. 3;
FIG. 11 is a flowchart illustrating a process for determining the pattern of FIG. 5 to which time-series data corresponds;
FIG. 12 is a diagram illustrating determination of the transition patterns;
FIG. 13 is an enlarged view of an example of the initial picture shown in FIG. 3;
FIG. 14 is a diagram showing an example in which a moving-average graph for the last five days is displayed in a "daily life data" column on the picture of FIG. 13;
FIG. 15 is a diagram showing an example of a display picture of the display means displayed before the third graph analysis of FIG. 10 is made;
FIG. 16 is a diagram showing an example of the display picture of the display means displayed after the third graph analysis of FIG. 10 is made;
FIG. 17 is a diagram illustrating the way of setting the data type and analysis section on the picture of FIG. 15 by means of a mouse cursor;
FIG. 18 is shows an example of display as a result of the graph analysis with the data type and analysis section set on the picture of FIG. 17 by means of the mouse cursor; and
FIG. 19 is a block diagram of a personal computer having a function as the healthcare apparatus according to the present invention.

### BEST MODE FOR CARRYING OUT THE INVENTION

A healthcare apparatus according to the present invention comprises input means for inputting a plurality of types of data that are used for a subject's healthcare or diagnosis, storage means for storing these data, and analyzing means for analyzing the correlation between the data. The analyzing means analyzes the correlation between the data by determining a corresponding transition pattern of the data, out of a plurality of transition patterns, of which the transition with time is predetermined.

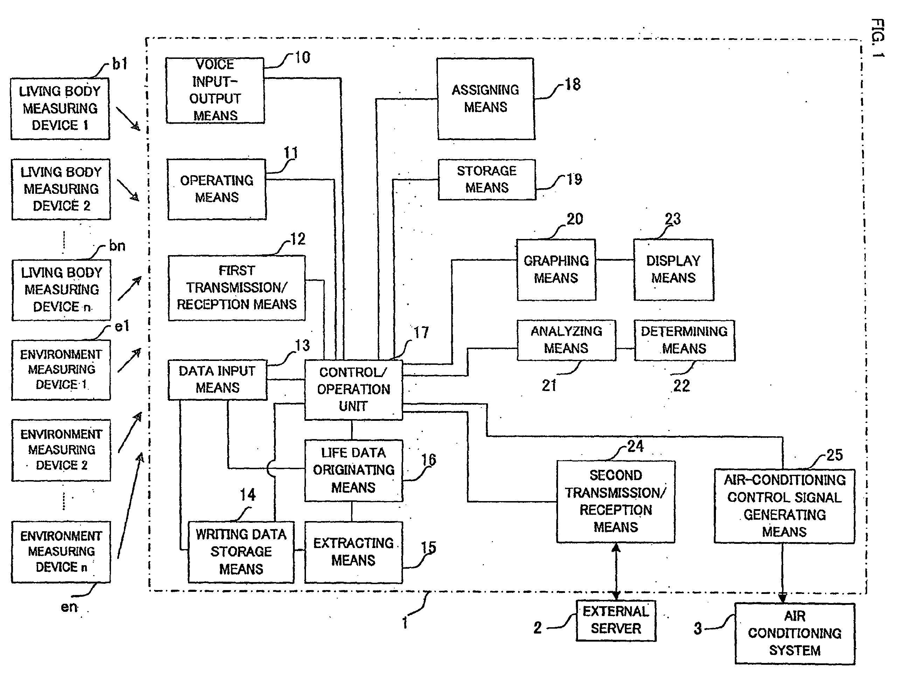

One embodiment of the healthcare apparatus according to the present invention will first be described with reference to FIG. 1.

A healthcare apparatus 1 comprises a control/operation unit 17 that is composed of a processor for controlling the entire apparatus. The control/operation unit 17 is connected with voice input-output means 10, operating means 11 such as a mouse, first and second transmission/reception means 12 and 24, data input means 13, writing data storage means 14, life data originating means 16, assigning means 18, storage means 19, graphing means 20, air-conditioning control signal generating means 25, etc. by means of buses.

As mentioned later, a voice message (vocal health diary) related to the physical condition, lifestyle, mood, etc. is inputted through the voice input-output means 10, and the message data is loaded into the writing data storage means 14. Health diary data that is inputted through a keyboard or the like of the data input means 13 is also stored in the writing data storage'means 14. Extracting means 15 extracts words that are predetermined, grouped, and weighed from the health diary data that are stored in the writing data storage means 14, and the life data originating means 16 weighs and quantifies the extracted words. The life data originating means 16 also quantifies data that are inputted in an inquiry form through the data input means 13.

The healthcare apparatus 1 is connected with living body measuring devices b1 to bn through the first transmission/reception means 12 that constitutes means for inputting data. The measuring devices b1 to bn measure the bodily temperature, weight, body fat percentage, blood pressure, pulse rate, etc. by utilizing a radio communication system or the like. Further, the healthcare apparatus 1 is connected with environment measuring devices e1 to en for the temperature, humidity, etc. of the ambiance of the subject.

The storage means 19 stores the data originated by means of the aforesaid living body measuring devices b1 to bn, environment measuring devices e1 to en, and life data originating means 16 for a given period (e.g., last three months). The assigning means 18 is means for inputting commands for the display, analysis, etc. of various data obtained in this manner.

Display means 23 is connected to the graphing means 20. Further, determining means 22 is connected to analyzing means 21. The analyzing means 21 analyzes the correspondence between the transition of the data and a plurality of predetermined patterns. The determining means 22 determines whether the pattern of the data analyzed'by means of the analyzing means 21 is identical with or contrary to an assigned pattern.

As mentioned later, the analyzing means 21 and the determining means 22 cooperate to determine the correspondence between the transition of the data inputted through the input means 13 with time and a plurality of predetermined transition patterns, thereby analyzing the correlation between the input data.

Further, the second transmission/reception means 24 is connected to an external server 2, such as a center that controls the healthcare apparatus 1 or a caregiving center for the subject, by means of a communication line. Furthermore, the air-conditioning control signal generating means 25 is connected to an air conditioning system 3 for a space in which the subject dwells.

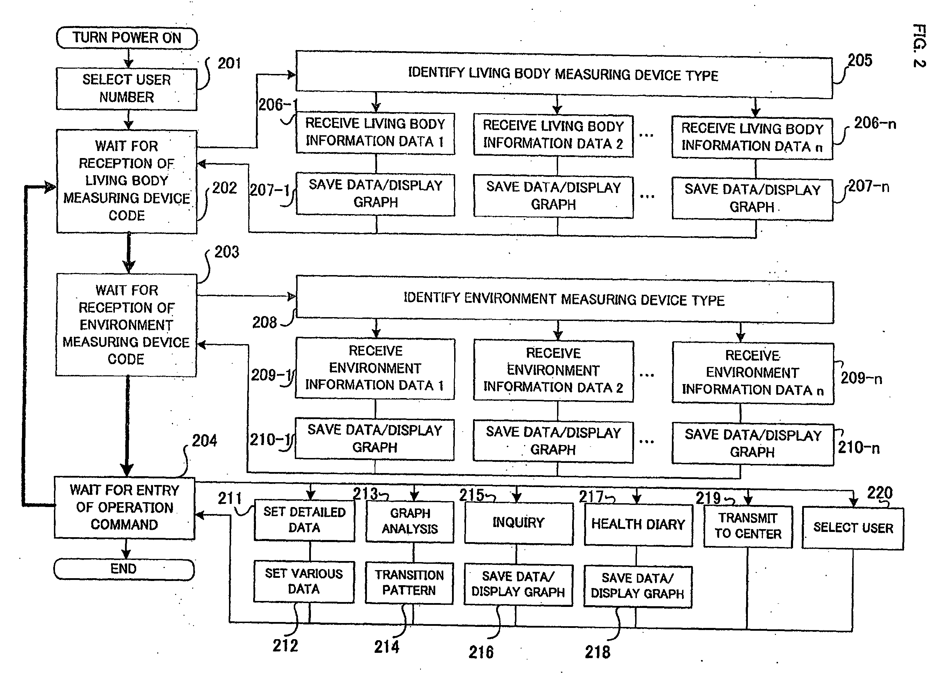

The principal operation of the healthcare apparatus shown in FIG. 1 will now be described with reference to FIG. 2.

If the user number is selected after the healthcare apparatus 1 is switched on, the processor of the control/operation unit 17 repeatedly determines whether or not identification codes (hereinafter referred to as codes) are received from the living body measuring devices b1 to bn and the environment measuring devices e1 to en through the first transmission/reception means 12 (202 and 203) and whether or not operation commands are inputted through the voice input-output means 10, operating means 11, data input means 13, and assigning means 18 (204).

If the subject switches on the healthcare apparatus 1 and inputs a transmission command, for example, the living body measuring devices b1 to bn transmit the then measurement data, along with codes indicative of the living body measuring devices, to the healthcare apparatus 1. If the subject inputs the transmission command, moreover, the environment measuring devices e1 to en also transmit measurement data, along with codes indicative of the environment measuring devices e1 to en. Alternatively, the measurement data may be transmitted for a given time or with every given time. If the healthcare apparatus 1 receives a code from any of the living body measuring devices b1 to bn, it identifies the type of the living body measuring device by that code (205), and receives measured living body data 1 to n (206-1 to 206-n). The data are saved in the storage means 19 for each user number, and the saved data are graphed by the graphing means 20 and displayed on the display means 23 (207-1 to 207-n).

If the healthcare apparatus 1 receives the measurement data, along with the codes indicative of the environment measuring devices e1 to en, from any of the environment measuring devices (203), on the other hand, it identifies the type of the environment measuring devices e1 to en by the code (208), and receives measured environment data 1 to n (209-1 to 209-n). The data are saved in the storage means 19, and the saved data are graphed by the graphing means 20 and displayed on the display means 23 (210-1 to 210-n).

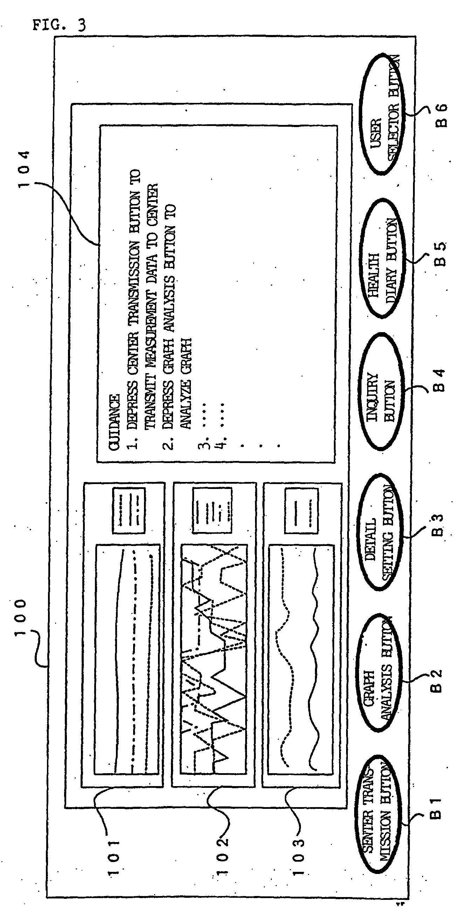

If the user number is selected after the healthcare apparatus 1 is switched on, an initial picture is displayed on a display screen 100 of the display means 23, as shown in FIG. 3. Up-to-date data for the last one month, among the data stored in the storage means 19, are displayed on a living body data column 101, daily life data column 102, and environment data column 103 of the initial screen. A guidance message for button operation or the like is displayed in a guidance column 104. Further, a command button B1 for transmitting data to the center, button B2 for instructing graph analysis, button B3 for setting detailed data for analysis, button B4 for inquiry, button B5 for inputting the health diary, and a user selector button B6 are displayed on the display screen 100. These buttons for the various commands displayed on the display screen 100 of the display means 23 constitute the aforementioned assigning means 18. The commands are given as the command buttons B1 to B6 are clicked by means of the mouse of the operating means 11.

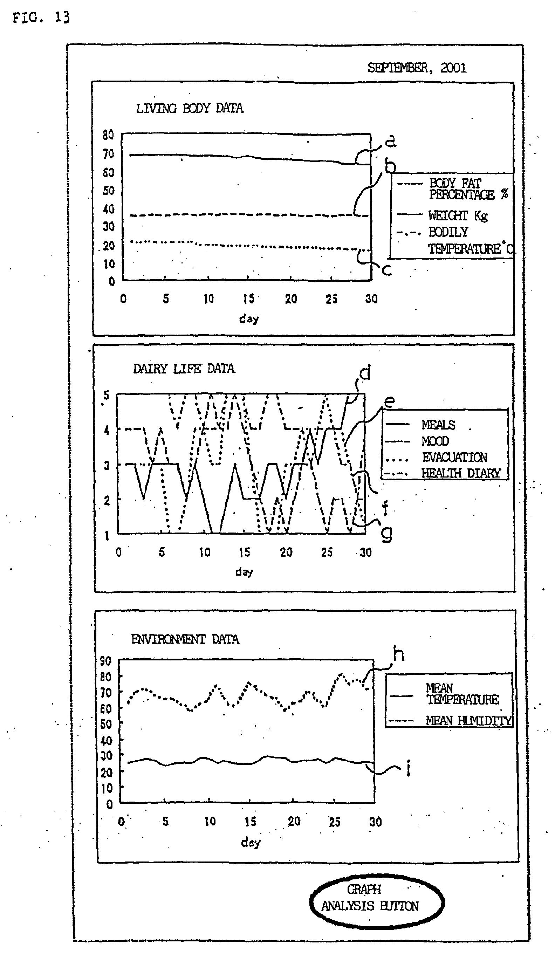

FIG. 13 is an enlarged view of the living body data column 101, daily life data column 102, and environment data column 103 in the initial picture shown in FIG. 3, and shows an example of display of the measured storage data in the columns 101, 102 and 103. In this example, the weight, bodily temperature, and body fat percentage are represented as the living body data by a full-line graph a, dashed-line graph b, and broken-line graph c, respectively. As graphs for daily life data, a full-line graph d, fine-broken-line graph e, dashed-line graph f, and rough-broken-line graph g represent meals, evacuation, health diary, and mood, respectively. As environment data, moreover, the mean temperature and mean humidity are represented by a full-line graph i and a broken-line graph h, respectively. Full lines, broken lines, and dashed lines are used for discriminative indication in order to discriminate the types of data of the graphs. Alternatively, however, classification by coloring may be used for the display.

If the center transmission button B1 is clicked, its operation command is detected (204). The processor of the control/operation unit 17 transmits the living body data, daily life data, and environment data to be stored in the storage means 19 to a host computer of the center that controls the healthcare apparatus 1 through the second transmission/reception means 24 (219).

If the graph analysis button B2 is clicked, moreover, various graph analyses are made (213), and a tendency pattern is obtained (214), which will be mentioned later.

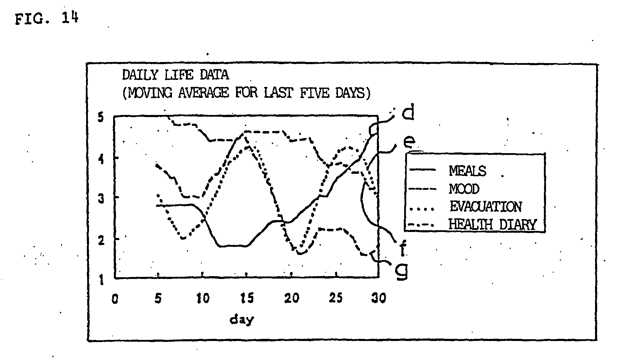

If the detail setting button B3 is clicked, various processes to be executed in the healthcare apparatus 1 can be set (212). Since the daily life data are discrete and discontinuous, in particular, they are represented by graphs such that the state of daily life cannot be grasped with ease, as shown in FIG. 13. According to this embodiment, a moving-average graph for the last five days can be set by means of the detail setting button B3. If the moving average for the last five days is selected and set, the moving average for the last five days is processed for the daily life data, as shown in FIG. 14. Thereupon, the moving-average graph for the last five days is displayed in the daily life data column 102, as shown in FIG. 14. In contrast with this, the original data may be selected and displayed as shown in FIG. 13 in place of the moving-average graph for the last five days.

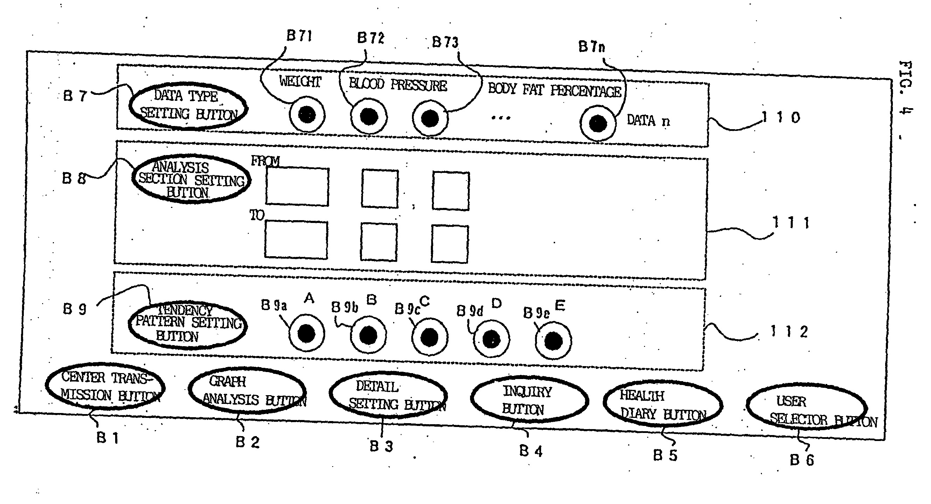

If the detail setting button B3 is clicked to select an analysis setting picture, moreover, the analysis setting picture is displayed on the display screen 100 of the display means 23, as shown in FIG. 4 (212). Various setting for the graph analyses can be effected by referring to the screen shown in FIG. 4. This analysis setting picture is used to set conditions for the graph analyses, and can carry a data type setting column 110 for setting data to be analyzed, an analysis section setting column 111 for setting an analysis section, and a pattern setting column 112 for analyzing a tendency pattern, which will be mentioned later.

The data type setting column 110 is provided with data selector buttons B71 to B7n and data type setting button B7. If the data type setting button B7 is clicked after the data selector buttons B71 to B7n are clicked to select the data to be analyzed, the selected data are analyzed in the manner mentioned later. Alternatively, the data type may be selected by means of the mouse, as mentioned later.

If an analysis section setting button B8 is clicked after the analysis section is set by entering starting and ending dates for analysis in the analysis section setting column 111 by means of the keyboard or the like, furthermore, a graph analysis for the set section is made. In this case, the analysis section may be also assigned by means of the mouse, as mentioned later.

Five pattern selector buttons B9a, B9b, B9c, B9d and B9e and a tendency pattern setting button B9 are displayed in the pattern setting column 112. In this embodiment, the time-based transition of the measured stored data is classified into five patterns. If the pattern selector buttons B9a to B9e are clicked for selection and if the tendency pattern setting button B9 is clicked, a selected pattern is selected and set, and data related to the selected pattern are selected and analyzed.

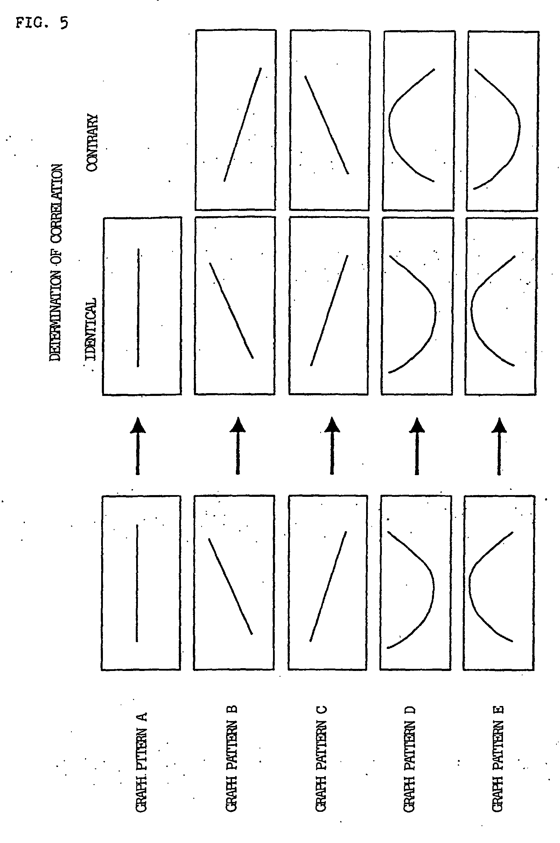

FIG. 5 is a diagram illustrating the five patterns. A pattern A indicates that the measurement data values maintain a substantially fixed tendency without changing with time. A pattern B indicates that the values of the measurement data have a tendency to increase with time. A pattern C indicates that the values of the measurement data values have a tendency to decrease with time. Thus, the patterns B and C are patterns that have tendencies contrary to each other. Further, a pattern D is a downwardly convex pattern that indicates the measurement data in a rectangular coordinate system having an axis of abscissa that represents time. A pattern E is an upwardly convex pattern that indicates the measurement data in a rectangular coordinate system having an axis of abscissa that represents time. Thus, the patterns D and E are patterns that have tendencies contrary to each other.

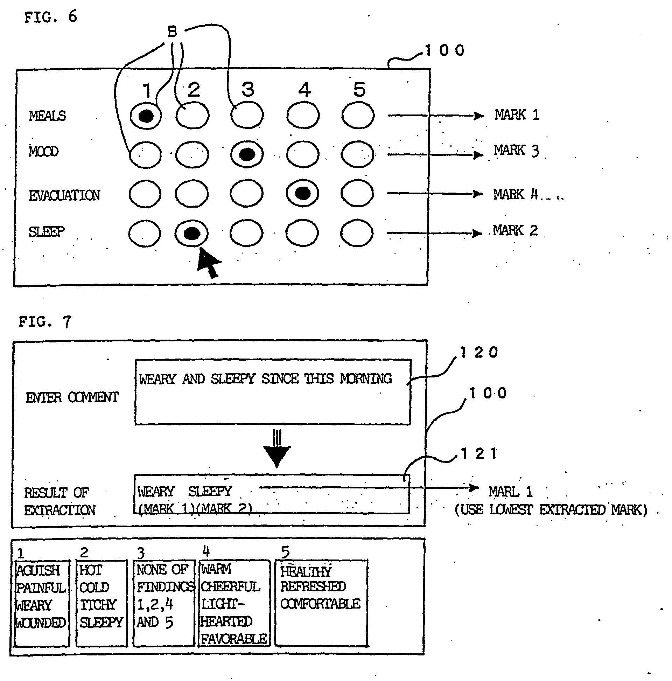

If the inquiry button B4 is clicked, an inquiry picture such as the one shown in FIG. 6 is displayed on the display means 23 (215). Although the command buttons B1 to B6 are also displayed, they are not shown in FIG. 6. In the example shown in FIG. 6, "meals", "mood", "evacuation", and "sleep" are selected as items that represent the state of daily life. The buttons B are displayed individually for five stages 1 to 5 of evaluation on each item. After the picture shown in FIG. 6 is displayed, the individual items are evaluated, and their corresponding buttons B are operated for entry by clicking or the like by means of the mouse or a cursor. For the item "meals", for example, the healthfulness of each meal is evaluated in five stages. The button B for "1" is selected for entry for a very poor meal; "2" for a poor meal, "3" for a passable meal, "4" for a healthful meal, and "5" for a very healthful meal. A numerical value corresponding to the selected button gives a mark for the item concerned.

For the item "mood", "1" is selected likewise for entry for a very unwell mood that implies a lot of worries, "5" for a very well refreshed mood, etc. The same applies to "evacuation" and "sleep", that is, the buttons B corresponding to lower marks are selected for entry for worse cases, and the buttons B corresponding to higher marks for better cases. FIG. 6 shows the case where the button B for 1 is selected to give the mark 1 to "meals", button B for 3 to give the mark 3 to "mood", button B for 4 to give the mark 4 to "evacuation", and button B for 2 to give the mark 2 to "sleep". The data inputted in this manner are saved as the daily life data by being loaded into the storage means 19. Then, the initial picture is restored, whereupon the new input data are added for graphic display (216).

If the health diary button B5 is then clicked, the picture 100 shown in FIG. 7 is displayed on the display means 23 (217). This picture has a comment input column 120 and a column 121 that displays the result of extraction of words from a comment. Thus, the comment is inputted by a voice through the voice input-output means 10 or through the keyboard or the like of the data input means 13. This comment is displayed in the comment input column 120 and stored in the writing data storage means 14. A plurality of comments may be inputted. In this case, the writing data storage means 14 is stored with a plurality of inputted comment data, that is, a plurality of writing data. The extracting means 15 extracts weighing words from the writing data stored in the writing data storage means 14. The extracted words are displayed in the display column 121, and a minimum weight of the words that are extracted by the life data originating means 16 is selected as the mark of the health diary. The mark for the health diary inputted in this manner is loaded into the storage means 19, and the initial picture is restored, whereupon the new input data are added for graphic display (216).

FIG. 7 shows an example of the weight on the words. Thus, lower marks are given to those words which are used to describe physically or mentally unhealthful states, while higher marks to those words which are used to describe physically or mentally healthful states. These words are weighted in five stages and evaluated.

Further, the user selector button B6 is expected to be operated by a subject, different from the subject who inputs the user number when the power is turned on (see numeral 201), in order to select his/her own user number when he/she uses the healthcare apparatus 1 that has already been switched on. If the user selector button B6 is operated to select the new user number (220), living body data and daily life data that are stored corresponding to that user number are read and displayed, and environment data are also displayed.

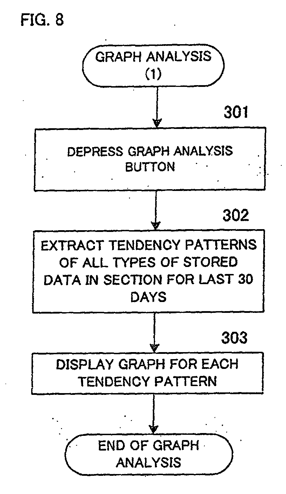

The following is a description of the graph analyses. Three kinds of graph analyses are provided according to the present embodiment. First, a first graph analysis will now be described with reference to FIG. 8.

If the graph analysis button B2 is clicked (Step 301), the processor of the control/operation unit 17 reads out the data stored in the storage means 19 for the last 30 days, and determines the correspondence between the tendency pattern of the data in this section and each of the aforesaid patterns A to E (Step 302). Then, the data that is concluded to have any of the patterns A to E is displayed for each pattern on the display screen of the display means 23 (Step 303). If the tendency pattern of the data is concluded to be the pattern B, for example, only the graph for the data having the pattern B is displayed on the display screen. Alternatively, those patterns which are contrary to the other tendency patterns B to E than the tendency pattern A may be associated in advance with one another so that the graph of data having a pattern (pattern C) contrary to the discriminated pattern (pattern B) can be also displayed together on the same display screen. Displaying the graph that has the pattern contrary to the discriminated pattern is based on the ground that the ascending tendency of some data is attributable to the descending tendency of other data, in some cases. If the contrary pattern is also displayed, the respective graphs of the same pattern and the contrary pattern should be discriminated by different colors. When a plurality of types of data that belong to the same pattern are graphically displayed on the same screen, moreover, the types of the data can be discriminated by changing the respective display colors of the data types (graphs).

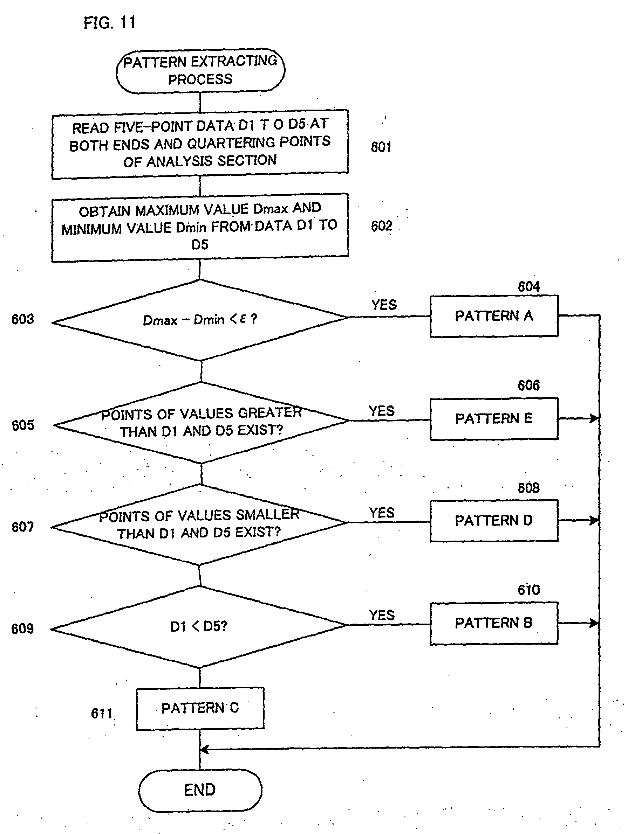

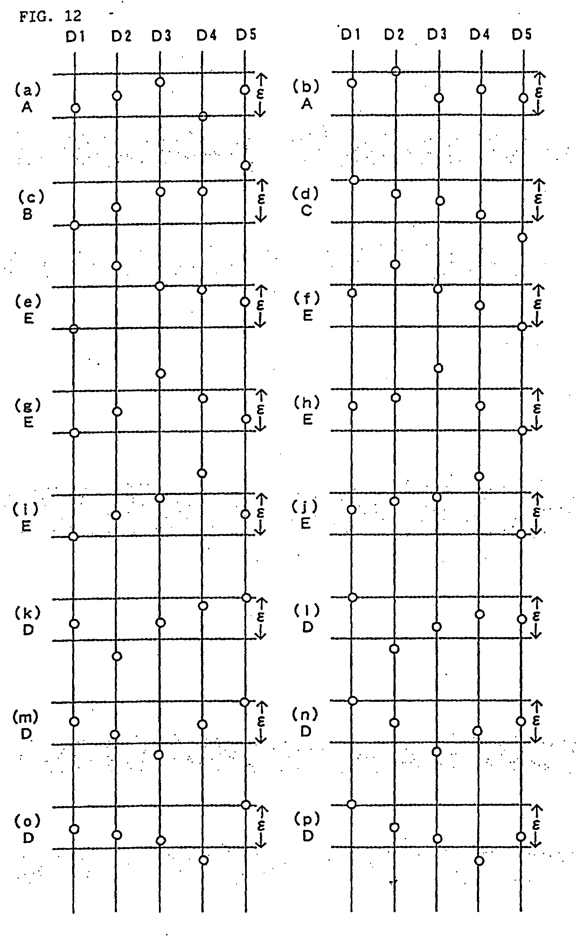

The determination of the tendency patterns of the data will now be described with reference to FIGS. 11 and 12.

First, both ends of a section to be analyzed and three dividing points that divide the section into four subsections of substantially equal width are obtained, and data values D1 to D5 are read out (Step 601). D1 is the value of data for the first day of the section, D2, D3 and D4 are the respective values of data for the dates of the dividing points in chronological order, and D5 is the value of data for the date of termination of the section. Then, maximum and minimum values of the data are obtained as Dmax and Dmin (Step 602), and whether or not the difference between the maximum and minimum values Dmax and Dmin is smaller than a given value ε (Step 603). If the difference is smaller, it is concluded that the pattern is the pattern A (Step 604) .

If the difference between the maximum and minimum values Dmax and Dmin of the data D1 to D5 is smaller than the given value ε, as shown in FIGS. 12(a) and 12(b), it is concluded that the pattern is the pattern A. In FIGS. 12(a) and 12(b), D1 to D5 on lines indicate that the data are situated in the respective positions of circles on the individual lines.

If it is concluded in Step 603 that the difference between the maximum and minimum values Dmax and Dmin exceeds the given value ε, whether or not data of values greater than the data values D1 and D5 for the dates at the opposite ends of the set section exist in D2, D3 and D4 is determined (Step 605). Thus, if there are data of values greater than the data D1 and D5 at the opposite ends of the section, as shown in FIGS. 12(e) to 12(j), it is concluded that the pattern is the pattern E, an upwardly convex pattern (Step 606).

If the decision in Step 605 is "No", whether or not data of values smaller than the data values D1 and D5 for the dates at the opposite ends of the set section exist in D2, D3 and D4 is determined (Step 607). Thus, if there are data of values smaller than the data D1 and D5 at the opposite ends of the section, as shown in FIGS. 12(k) to 12(p), it is concluded that the pattern is the pattern D, a downwardly convex pattern (Step 608).

If the decisions in Steps 603, 605 and 607 are "No", that is, if the difference between the maximum and minimum values Dmax and Dmin exceeds the given value ε and if no data that are greater or smaller than the data values D1 and D5 for the dates at the opposite ends of the set section exist in the data D2, D3 and D4 at the intermediate dividing points, then whether or not the data value D5 for the last day of the section is greater than the data value D1 for the first day of the section is determined (Step 609). If the value D5 is greater, that is, if the state shown in FIG. 12(c) is established, the pattern is concluded to be the pattern B (Step 610). If the state shown in FIG. 12(d) is established, in contrast with this, the pattern is concluded to be the pattern C (Step 611).

Although the pattern extracting process according to the present embodiment has been described above, the pattern may be discriminated with reference to three data, for example. Alternatively, these data may be increased so that the pattern can be discriminated more accurately with use of a higher criterion of discrimination.

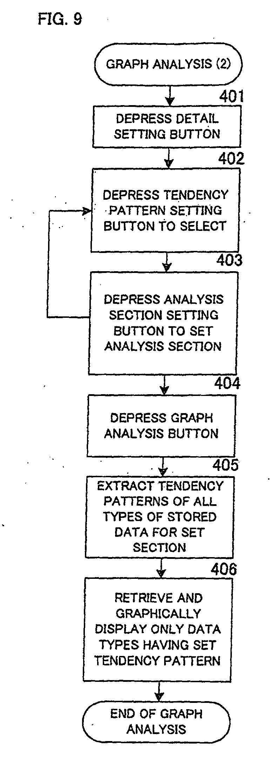

A second graph analysis will now be described with reference to FIG. 9.

The detail setting button B3 is clicked to select and display the analysis setting picture shown in FIG. 4 (Step 401), and any of the selector buttons B9a to B9e for the patterns A to E is clicked to select the pattern. Thereafter, the tendency pattern setting button B9 is clicked to set the selected pattern (Step 402). Further, the starting and ending dates of the analysis section are entered in the section setting column 111 of the picture, and the analysis section setting button B8 is clicked (Step 403). The processes of Step 402 and 403 for pattern selection setting and section setting may be executed in random order.

If the graph analysis button B2 is depressed after the pattern and the section are set, the processes shown in the flowchart of FIG. 11 are executed for each type of data to cover all types of data that are stored in the storage means 19, and the tendency pattern for the set section is obtained (Step 405). Only the data having the tendency pattern selected and set in Step 402 are extracted and graphically displayed (Step 406).

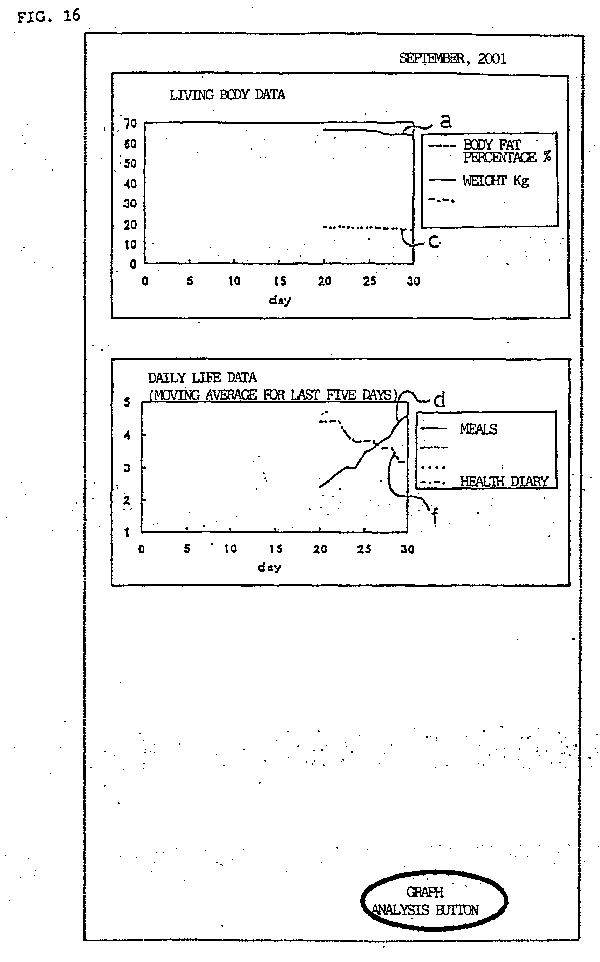

When the analysis picture shown in FIG. 4 is displayed, for example, the selector button B9c is depressed to select the pattern C, and the analysis section is adjusted to the period between the twentieth and the thirtieth of one month. If the second graph analysis shown in FIG. 9 is made in this state, a weight graph a, body fat percentage graph c, and health diary graph f of the living body data having the pattern C are selected, and only these selected graphs (i.e., only data types having the same tendency) are displayed on the display screen 100 of the display means 23. This display state is obtained by omitting a meal graph d from the display picture of FIG. 16.

Thus, in this second graph analysis, the tendency pattern of the data of the data type corresponding to the set pattern is displayed in the set analysis section.

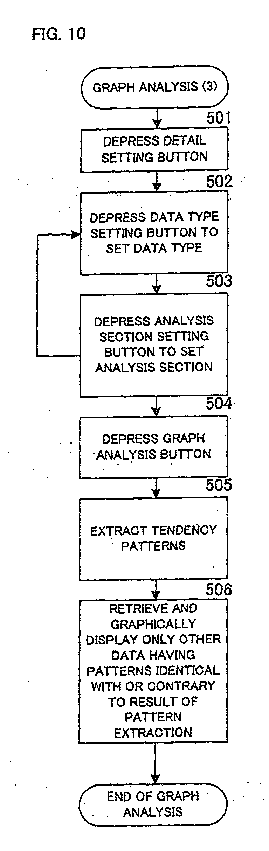

A third graph analysis will now be described with reference to FIG. 10.

The detail setting button B3 is clicked to display the analysis setting picture shown in FIG. 4 (Step 501), and any of the data selector buttons B71 to B7n is clicked to select the data type. Thereafter, the data type setting button B7 is clicked to select the data type (Step 502). Further, the starting and ending dates of the analysis section are entered in the section setting column 111, and the analysis section setting button B8 is clicked (Step 503). The data type selection in Step 502 and the analysis section setting in Step 503 may be executed in random order.

If the graph analysis button B2 is then depressed (Step 504), the process of FIG. 11 is executed for the section set in Step 503 to cover all the data types, and the tendency pattern for each data type is extracted (Step 505). Then, the data of the data pattern having the same pattern set in Step 502 and the data of another type having a contrary pattern are extracted and displayed (Step 506).

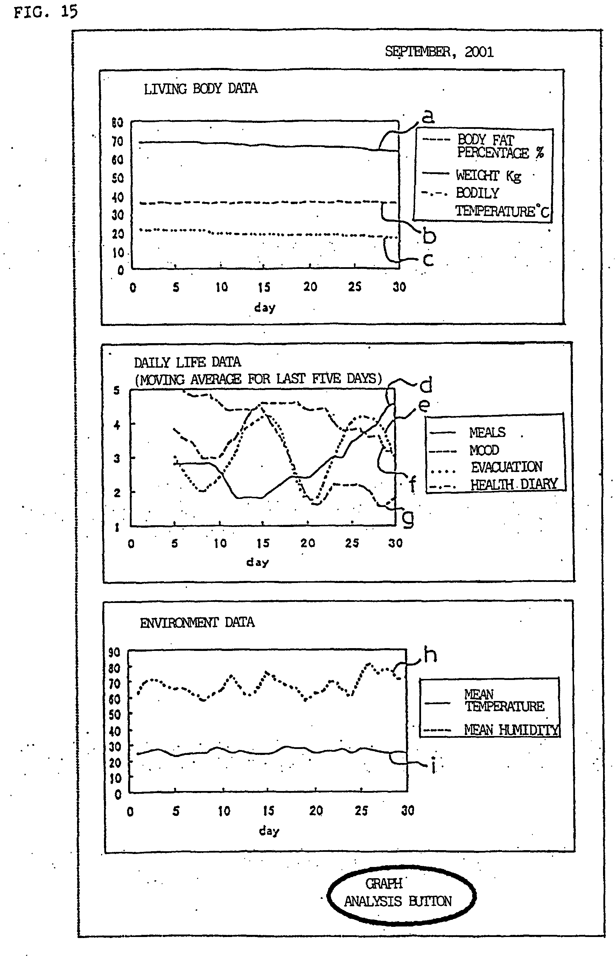

FIG. 15 shows an example of a display picture of the display means 23 displayed before the third graph analysis is made. In this state, the weight is selected as the data type (i.e., the data type setting button B7 in the picture of FIG. 4 is depressed after the data selector button B71 is depressed), and the period between the twentieth and the thirtieth of September, 2001 is set as the analysis section. If the third graph analysis is executed in this state, a graph a of weight data, graph c of body fat percentage data, graph f of health diary data, and graph d of meal data are displayed for the period between the twentieth and the thirtieth of September, 2001 in the analysis section, as shown in FIG. 16. The graphs c and f have the same pattern as the tendency pattern of weight (pattern C in the example of FIG. 13). The graph d has a pattern (pattern B) contrary to the aforesaid pattern.

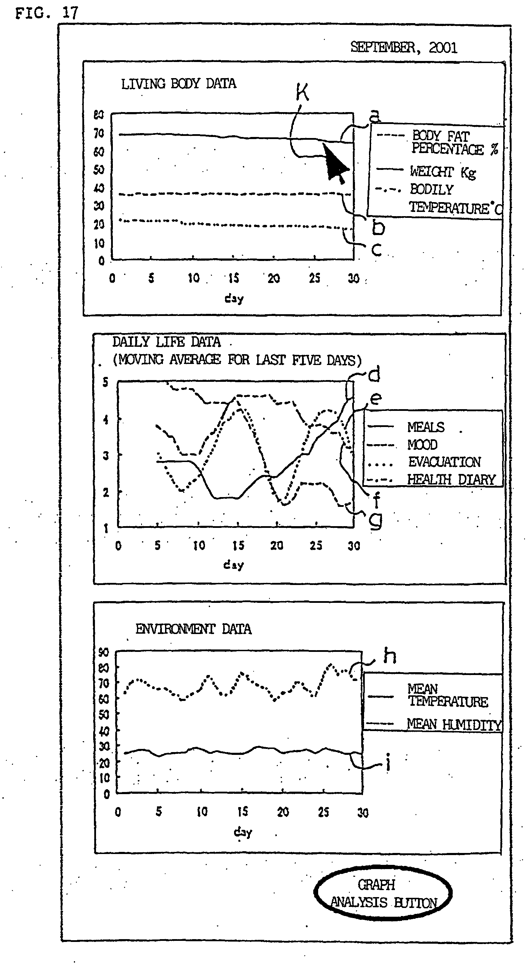

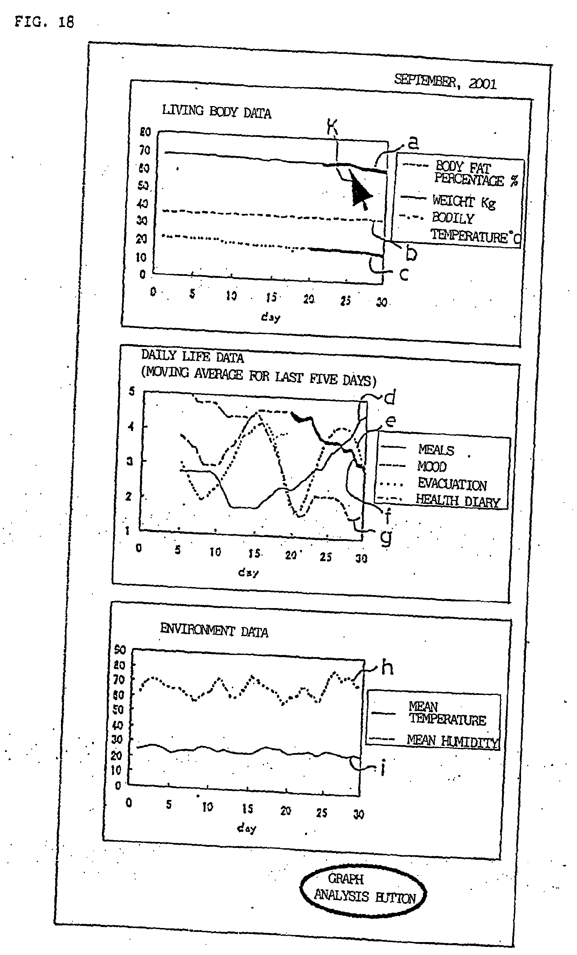

Alternatively, an analysis that is equivalent to the third graph analysis may be made by setting the data type and section by means of the mouse. An example of the graph analysis using the mouse will be described with reference to FIGS. 17 and 18.

The data type and section are set by tracing a graph a of weight data of the conventional graph display shown in FIG. 17 for the section between the twentieth and the thirtieth of September, 2001, for example, by means of a mouse cursor (assigning means) K. Then, a graph analysis is made by depressing the graph analysis button B2. FIG. 18 shows the result of the' graph analysis. As shown in FIGS. 17 and 18, the weight is reduced in the section from the twentieth to the thirtieth, assuming the pattern C. The body fat percentage and health diary are the data types of which the tendency in the section from the twentieth to the thirtieth has the pattern C. As shown in FIG. 18, therefore, the weight, body fat percentage, and health diary in the section from the twentieth to the thirtieth are exclusively indicated by thick lines (or lines of colors different from those of the other graphs). In this example, as described above, the data and the section are set by means of the cursor K, and only the data types having the same pattern as that of the set data are displayed in distinction from the others.

The data handled by the healthcare apparatus 1 according to the embodiment described above are of three types, living body data, environment data, and life data. If data of at least one type, out of the data the healthcare apparatus 1 handles, are living body data, change of the body condition can be recognized. If data of at least one type are environment data, moreover, change of the living environment that influences the body can be recognized. If data of at least one type are life data, change of physical condition the subject feels can be recognized.

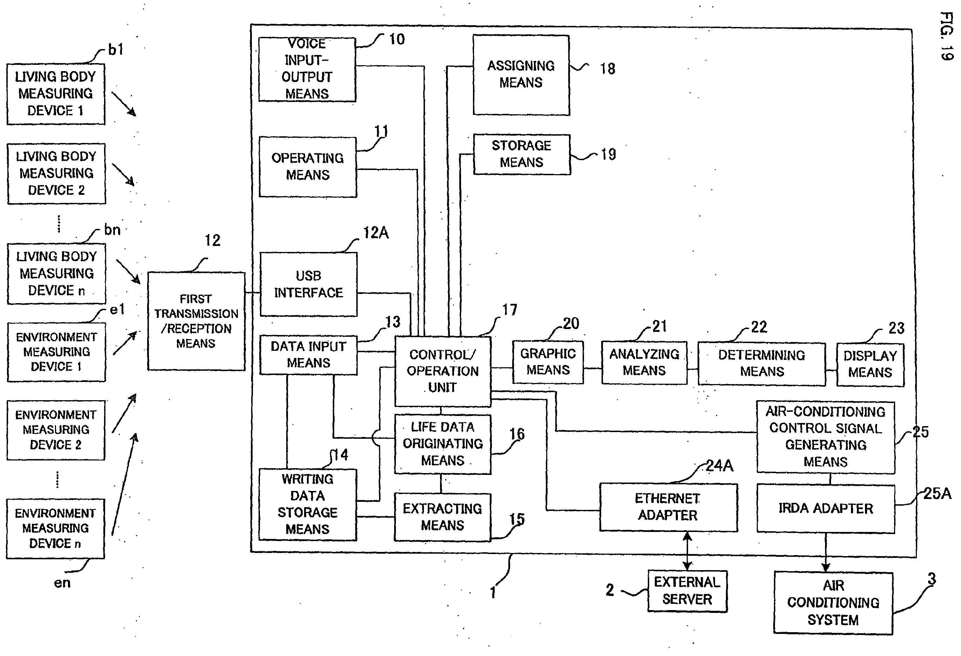

A personal computer can be made to bear the function of the healthcare apparatus 1 shown in FIG. 1. FIG. 19 shows this personal computer. In FIG. 19, elements that have the same numerals with the elements of FIG. 1 have the same functions.

In a personal computer 1A that functions as the healthcare apparatus according to the present invention, as shown in FIG. 19, its CPU serves as the control/operation unit 17; its keyboard as the data input means 13, its memories as the storage means 19 and the data storage means 14, and its display as the display means 23. Further, the respective functions of the life data originating means 16, extracting means 15, graphing means 20, analyzing means 21, determining means 22, and air-conditioning control signal generating means 25 are realized by programs that are stored in a floppy disc or CD-ROM, which will be mentioned later.

An external unit that functions as the first transmission/reception means 12 is connected to a USB interface 12A of the personal computer 1A. The living body measuring devices b1 to bn, environment measuring devices e1 to en, and personal computer 1A wirelessly communicate with one another by means of the first transmission/reception means 12. The USB interface 12A is a general-purpose interface that is incorporated in the personal computer and can be connected with various external apparatuses. If the 'external unit that constitutes the first transmission/reception means is connected to the USB interface, the personal computer can transmit to and receive data from the individual living body measuring devices.

Further, the personal computer 1A is provided with an Ethernet adapter 24A and an IrDA (Infrared DATA Association) adapter 25A. This Ethernet adapter is an adapter that is incorporated in or externally attached to the personal computer. It serves for the connection to the Internet and transmits to and receives data from the external server and the like. The Ethernet adapter 24A is connected to the external server 2, e.g., the center that controls the healthcare function of personal computer 1A, caregiving center for the subject, etc., by means of the communication line. Furthermore, the IrDA adapter is an adapter that is incorporated in or externally attached to the personal computer. It transmits to and receives data and control signals from an external apparatus; such as an air conditioning system, by means of infrared rays. The IrDA adapter 25A is connected to the air-conditioning control signal generating means 25. Thus, an air-conditioning control signal is generated in the air-conditioning control signal generating means 25 if it is concluded to be necessary by the control/operation unit 17 that analyzes the measurement data fetched from the living body measuring devices b1 to bn and the environment measuring devices e1 to en by means of the first transmission/reception means 12. Thus, the air conditioning system 3 for the space in which the subject dwells is controlled by infrared data communication.

Further, the data stored in the storage means 19 are read by means of the control/operation unit 17 and graphed by the graphing means 20. The analyzing means 21 analyzes the correspondence between the transition of the data and a plurality of predetermined patterns, and the determining means 22 determines whether the pattern analyzed by the analyzing means 21 is identical with or contrary to the assigned pattern. The result of the determination is displayed on the screen of the display means 23.

A recording medium, such as the floppy disc or CD-ROM, to be loaded into the personal computer 1A has the following contents.
(1) A computer-readable recording medium registered with a program that enables the computer to realize a function to receive data from a plurality of types of measuring devices that are used for the subject's healthcare or diagnosis, a function to store the data in the storage means, and a function to read the data stored in the storage means and analyze the presence of correlation between the data.
(2) A computer-readable recording medium registered with a program that enables the computer to realize a function to receive data from a plurality of types of measuring devices that are used for the subject's healthcare or diagnosis, a function to store the data in the storage means, and a function to read the data stored in the storage means and analyze the presence of correlation between the data by determining the correspondence between the transition of the data with time and a plurality of predetermined transition patterns.
(3) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data having a given transition pattern from said plurality of types of data.
(4) A computer-readable recording medium registered with a.program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data having a transition pattern selected from a plurality of predetermined patterns and assigned.
(5) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data of another type having the same transition pattern with data of one type when the data of the one type is assigned.
(6) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data of another type having the same transition pattern as that of the data of one type and data of a type having a transition pattern associated with the aforesaid transition pattern when the data of the one type is assigned.
(7) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data of another type having the same transition pattern as that of the data of one type and data of a type having a transition pattern having a tendency to contradict the aforesaid transition pattern when the data of the one type is assigned.
(8) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data having the same transition pattern as one transition pattern and data having a transition pattern associated with the one transition pattern when the one transition pattern, among the aforesaid plurality of patterns, is assigned.
(9) A computer-readable recording medium registered with a program that enables the computer to realize a function to receive data from a plurality of types of measuring devices that are used for the subject's healthcare or diagnosis, a function to store the data in the storage means, a function to read the data stored in the storage means and analyze the presence of correlation between the data by determining the correspondence between the transition of the data with time and a plurality of predetermined transition patterns, and a function to extract data having the same transition pattern as one transition pattern and data having a transition pattern having a tendency to contradict the one transition pattern when the one transition pattern, among the aforesaid plurality of patterns, is assigned.

## Claims

1. A healthcare apparatus comprising:
input means for inputting a plurality of types of data to be used for the healthcare or diagnosis of a subject;
storage means for storing the data; and
analyzing means for analyzing the presence of correlation between the data.

2. The health care apparatus according to claim 1, wherein the analyzing means analyzes the presence of correlation between the data by determining the correspondence between the transition of the data with time and a plurality of predetermined transition patterns.

3. The health care apparatus according to claim 2, wherein data having a given transition pattern are extracted from said plurality of types of data.

4. The health care apparatus according to claim 3, wherein the given transition pattern can be selected from a plurality of predetermined patterns and assigned.

5. The health care apparatus according to claim 2, wherein if data of one type is assigned, data of another type having the same transition pattern with the data of the assigned type is extracted.

6. The health care apparatus according 'to claim 2, wherein if data of one type is assigned, data of another type having the same transition pattern as that of the data of the assigned type and data of a type having a transition pattern associated with said transition pattern are extracted.

7. The health care apparatus according to claim 6, wherein the associated transition pattern is a transition pattern having a tendency to contradict the transition pattern of the data of the assigned type.

8. The health care apparatus according to claim 2, wherein if one transition pattern is assigned from among said plurality of patterns, data having the same transition pattern as the assigned transition pattern and data having a transition pattern associated with said transition pattern are extracted.

9. The health care apparatus according to claim 8, wherein the associated transition pattern is a transition pattern having a tendency to contradict the assigned transition pattern.

10. The health care apparatus according to any one of claims 1 to 7, wherein the data of at least one type, among the data of said plurality of types, is living body data obtained by measuring the state of the body of the subject.

11. The health care apparatus according to any one of claims 1 to 7, wherein the data of at least one type, among the data of said plurality of types, is environment data obtained by measuring the conditions of the living environment of the subject.

12. The health care apparatus according to any one of claims 1 to 7, wherein the data of at least one type, among the data of said plurality of types, is life data obtained by quantifying the physical condition and lifestyle of the subject.

13. A healthcare program for realizing a storage function to load a computer with data of a plurality of types used for a healthcare or diagnose of a subject and store the data of said plurality of types in a storage unit of the computer and an analyzing function to analyze the presence of correlation between the data stored in the storage unit.

14. The health care program according to claim 13, wherein the analyzing function analyzes the presence of correlation between the data by determining the correspondence between the transition of the data and a plurality of predetermined transition patterns.

15. The health care program according to claim 14, wherein the healthcare program realizes an extracting function to extract data having a given transition pattern from the data of said plurality of predetermined types.

16. The health care program according to claim 14, wherein the healthcare program further realizes a function to select the given transition pattern from a plurality of predetermined patterns by means of input means of the computer and assign the pattern.

17. The health care program according to claim 14, wherein the healthcare program further realizes a function to extract data of another type having the same transition pattern as that of the data of one type if the data of the one type is assigned by means of input means of the computer.

18. The health care program according to claim 14, wherein the healthcare program further extracts data of another type having the same transition pattern as that of the data of one type and data of a type having a transition pattern associated with said transition pattern if the data of the one type is assigned by means of input means of the computer.

19. The health care program according to claim 18, wherein the associated transition pattern is a transition pattern having a tendency to contradict the transition pattern of the data of the assigned type.

20. The health care apparatus according to claim 14, wherein the healthcare program realizes an extracting function to extract data having the same transition pattern as one transition pattern and data having a transition pattern associated with said transition pattern if the one transition pattern is assigned among said plurality of transition patterns.

21. The health care program according to claim 20, wherein the associated transition pattern is a transition pattern having a tendency to contradict the assigned transition pattern.

22. The health care program according to any one of claims 13 to 19, wherein the data of at least one type, among the data of said plurality of types, is living body data obtained by measuring the state of the body of the subject.

23. The health care program according to any one of claims 13 to 19, wherein the data of at least one type, among the data of said plurality of types, is environment data obtained by measuring the conditions of the living environment of the subject.

24. The health care program according to any one of claims 13 to 19, wherein the data of at least one type, among the data of said plurality of types, is life data obtained by quantifying the physical condition and lifestyle of the subject.
